# EUROPÄISCHE PATENTSCHRIFT

(11) **EP 1 634 625 B1**
(45) Veröffentlichungstag und Bekanntmachung des Hinweises auf die Patenterteilung: **02.07.2008**
(21) Anmeldenummer: 05090232.9
(22) Anmeldetag: 10.08.2005
(51) Int. Cl.: A61Q 19/08, A61K 8/67, A61K 8/97, A61K 8/29, A61K 8/68, A61K 8/64

(54) **Pigmenthaltiges kosmetisches Mittel mit Anti-Alterungswirkung für die Haut**
Pigmented cosmetic product having anti-ageing poperty for the skin
Composition cosmétique pigmentée contre des signes cutanés du vieillissement

(30) Priorität: 11.08.2004 DE 102004039631
(43) Veröffentlichungstag der Anmeldung: 15.03.2006
(73) Patentinhaber: COTY PRESTIGE LANCASTER GROUP GmbH, 55116 Mainz (DE)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- WO-A-99/66881
- WO-A-03/013463
- WO-A-03/015740
- US-A1- 2003 059 484
- US-A1- 2004 115 163

## Beschreibung

Die Erfindung betrifft ein kosmetisches Mittel, das eine faltenglättende Anti-Alterungswirkung für die Haut aufweist und pigmenthaltig ist.

Aus WO 03/063803 sind bioaktive kosmetische Ingredienzien bekannt, darunter auch Medicago sativa, die eine entzündungswidrige Wirkung und antiproteolytische Wirksamkeit haben sollen. Von Crithmum maritimum ist allgemeine eine verdauungsfördernde Wirkung bekannt sowie ein gewisser Vitamin C-Gehalt.

Die US 2003/0059484 beschreibt ein Mittel mit Anti-Alterungswirkung, das u.a. Alfalfa-Extrakt zusammen mit Vitamin A, dessen Ester oder eine Vielzahl weiterer Pflanzenextrakte oder Wirkstoffe wie Ceramide enthalten kann. Die beschriebenen Anwendertests zeigen jedoch nur Ergebnisse bis 28 Tage.

Der Erfindung liegt die Aufgabe zugrunde, ein kosmetisches Mittel zur Verringerung der Hautfalten für längere Zeiträume zu entwickeln. Eine weitere Aufgabe ist die Verringerung der Faltentiefe, insbesondere auch bei Mimikfalten.

Erfindungsgemäß bereitgestellt wird ein pigmenthaltiges kosmetisches Mittel mit Anti-Alterungswirkung für die Haut, das einen Wirkkomplex, Trägerstoffe, Hilfsstoffe und gegebenenfalls weitere Wirkstoffe umfaßt, wobei der Wirkkomplex enthält
0,01 bis 5 Gew-% in Liposome verkapseltes Retinol,
0,1 bis 2 Gew-% Retinylpalmitat,
0,1 bis 3 Gew-% eines kohlehydratreichen, wässrigen Alfalfa-Extraktes
0,1 bis 5 Gew-% eines Meerfenchel-Extraktes und
0,01 bis 5 Gew-% Schichtpigmente, ausgewählt unter Glimmer, beschichtet mit TiO₂/SiO₂ und einer weiteren TiO₂-Schicht; SiO₂₋Plättchen, beschichtet mit Rutil-TiO₂; und Gemischen davon,
   und den Rest zu 100 Gew-%, bezogen auf das Gesamtgewicht des Mittels, die Trägerstoffe, Hilfsstoffe und gegebenenfalls weiteren Wirkstoffe bilden. Dabei sind alle Prozentangaben auf das Gesamtgewicht des kosmetischen Mittels bezogen

Es wurde gefunden, dass kosmetische Mittel mit dem erfindungsgemäßen Komplex als Wirkstoff sowohl die Anzahl feiner Hautfalten verringert als auch die Tiefe stärkerer Falten deutlich abschwächt, und diese Wirkung über längere Zeiträume beibehalten wird. Vergleichsversuche haben gezeigt, dass die Wirkung bei 4-wöchiger Anwendung z.B. als Gesichtscreme deutlich erkennbar ist und über einen Zeitraum von weiteren 4 Wochen anhält. Langzeittests über 8-12 Wochen zeigen eine mittlere Faltenreduzierung von 30 % und maximale Faltenreduzierungen von 50 %.

Besonders eindrucksvoll und überraschend ist die Langzeitwirkung ohne Weiterbehandlung, d.h. nach z.B. 8-wöchiger Behandlung zweimal täglich ist eine mittlere Faltenglättung von 30 % noch weitere 8 Wochen danach mit ca. 20-25 % erkennbar, wie Anwender-Tests ergeben haben.

Der erfindungsgemäße Wirkkomplex ist kein Komplex im rein chemischen Sinne, sondern ein Gemisch verschiedener Bestandteile, deren Zusammenwirken eine bisher nicht bekannte und nicht vorhersehbare Wirkung oder einen solchen Aspekt einer Wirkung zeigt. Der Komplex geht damit über die Summe der Einzelwirkungen der Bestandteile hinaus.

Der Meerfenchelextrakt wird durch Extraktion der Gesamtpflanze mit überkritischem CO₂ (31 °C, 74 bar) gewonnen und zeigt in erster Linie Wirksamkeit auf die Keratinocyten (Rezeptor CRABP und Cytokeratine CK1, CK6, CK10) und die Bildung von Transglutaminase, Loricrin und Involucrin.

Der Alfalfaextrakt beeinflusst signifikant die Keratinocytendifferenzierung und stimuliert die Synthese der Cytokeratine CK4 und CK19. Der kohlenhydratreiche wässrige Extrakt der Gesamtpflanze enthält Galactose, Mannose und Glucose und ist pH-stabil von pH 2 bis pH 10 auch im höheren Temperaturbereich von 40-80 °C.

Eine Verstärkung der Gesamtwirkung kann erreicht werden, wenn der erfindungsgemäße Wirkkomplex zusätzlich 0,1 bis 2 Gew-% eines Dattelpalmenkernextraktes und/oder 0,01 bis 5 Gew-% einer synthetisch hergestellten Assoziation von Ceramid 2 mit dem palmitoylierten Oligopeptid Matrikin (Pal-Val-Gly-Val-Ala-Pro-Gly) enthält. Die letztgenannte Assoziation stimuliert die genetische Expression von Granulocyten-chemotaktischem Protein (GCP-2) und trägt ebenfalls zur Faltenreduzierung bei und intensiviert überraschend die Langzeitwirkung. Bevorzugt werden davon 0,5 bis 2 Gew-% eingesetzt. Dabei sind alle Prozentangaben auf das Gesamtgewicht des kosmetischen Mittels bezogen.

Der Extrakt der Kerne von Dattelpalmfrüchten (Phoenix dactylifera) kann gemäß WO 03/013463 zur Verringerung von Hautfalten und zur Hautglättung eingesetzt werden. Ein solcher Extrakt enthält die Phytosterole Stigmasterol und Sitosterol, weiterhin Phytosteroide mit hormonähnlicher Wirkung, das Triterperioid Ursolsäure sowie Isoflavone, Tocotrienole und β-Caroten als Provitamin A. Die Extraktion erfolgt z.B. mit Chloroform/Methanol.

Die erfindungsgemäß einzusetzenden Pigmente zeigen neben dem besonderen Glättungseffekt auch einen farblichen Effekt, der in Abhängigkeit vom Betrachtungswinkel von senkrecht bis nahezu horizontal bei dem Pigment auf Glimmerbasis von rot über kupfer bis zu gold mit leichtem Grünschimmer verläuft und bei dem Pigment auf Basis SiO₂-Plättchen von grün über silber bis zu pfirsich verläuft. Die Teilchengröße der Pigmente liegt im Bereich von 5 bis 60 µm.

Bevorzugte Bereiche des erfindungsgemäßen Komplexes liegen bei 0,05 bis 1 Gew-% für das in Liposome verkapselte Retinol,
0,1 bis 0,9 Gew-% für das Retinylpalmitat,
0,5 bis 2 Gew-% für den Alfalfa-Extrakt,
1 bis 5 Gew-% für den Meerfenchel-Extrakt und
0,5 bis 3 Gew-% für die Schichtpigmente.

Der erfindungsgemäße Wirkstoffkomplex, der gegebenenfalls durch die zuvor genannten Zusätze erweitert werden kann, kann in bestimmten kosmetischen Anwendungsformen eingesetzt werden. Derartige Anwendungsformen sind z.B. Lotion, Gel, Tagescreme, Nachtcreme, Sonnencreme, Sonnenmilch, After-sun Produkt, Spray, Balm, Maske, Makeup, Handcreme, Körperpflegeprodukt und /oder Haarprodukt.

Die entsprechende Anwendungsform enthält neben dem Wirkkomplex kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Es können auch weitere zusätzliche Wirkstoffe neben dem Wirkkomplex eingesetzt werden. Zu diesen kosmetischen Wirkstoffen gehören z.B. anorganische und organische Lichtschutzmittel, Radikalfänger, Feuchthaltemittel, Vitamine, Enzyme, weitere pflanzliche Wirkstoffe, Polymere, Antioxidationsmittel, weitere entzündungswidrige natürliche Wirkstoffe, mit Sauerstoff beladene asymmetrische lamellare Aggregate gemäß WO 94/00109, Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu Antioxidationsmitteln gehören Vitamine wie Vitamin C und Derivate davon, beispielsweise Ascorbylacetate, -phosphate und - palmitate; Vitamin A und Derivate davon; Folsäure und deren Derivate, Vitamin E und deren Derivate, wie Tocopherylacetat; Superoxiddismutase; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure.

Es ist weiterhin vorteilhaft, den kosmetischen Zusammensetzungen mit dem erfindungsgemäßen Wirkkomplex entsprechende wasser-und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na-oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bos-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Besonders bevorzugt als anorganische Pigmente sind agglomerierte Substrate von TiO₂ und/oder ZnO, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden (gemäß WO99/06012).

Ein weiterer bevorzugter Wirkstoffzusatz für das erfindungsgemäße Kosmetikum ist eine Zubereitung mit hohem Radikalschutzfaktor (siehe WO99/66881) mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser. Diese Zubereitung kann gegebenenfalls noch ergänzt sein durch ein Superoxiddismutase-haltiges Hefeaufschlussprodukt und/oder Cyclodextrine (WO 94/13783).

Die für die Erfindung eingesetzten Öle können übliche kosmetische Öle sein, wie ein Mineralöl; hydriertes Polyisobuten , synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether sowie pflanzliche Öle, wie Calendulaöl, Jojobaöl, Avocadoöl, Macadamianußöl, Rizinusöl, Kakaoobutter, Kokosnußoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflußt, wie Transparenzgrad, Weichheit, Härte, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate, Propylene glycol dicaprylate 2,30 dicaprate, Tridecyl stearate/neopentyl glycol dicaprylate dicaprate/tridecyl trimellitate, Neopentyl glycol dioctanoate, Isopropyl myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure etc.

Als Feuchthaltemittel sind bevorzugt Glycerin, Butylenglycol, Propylenglycol und Gemische davon.

Für die Emulsionsbildung können als oberflächenaktive Mittel anionische, amphotere, nichtionische oder kationische oberflächenaktive Mittel oder Gemische davon eingesetzt werden.

Die Erfindung soll nachstehend durch Beispiele näher erläutert werden. Alle Angaben erfolgen in Gewichtsprozent, sofern nichts anderes angegeben ist.

### Beispiel 1 Gesichtscreme I

| **Phase A** | |
|---|---|
| Wasser | q.s. ad 100 |
| Glycerin | 4,0 |
| Propylenglycol | 2,0 |
| Pigmentgemisch Glimmerpigment und SiO₂-Pigment 1:1 | 3,0 |

| **Phase B** | |
|---|---|
| Dimethicone & Dimethicone Crosspolymer | 3,0 |
| Dimethicone & Cyclotetrasiloxane | 1,5 |
| Octyldecanol | 3,5 |

| **Phase C** | |
|---|---|
| Wirkstoffkomplex bestehend aus Retinol (in Liposomen) 0,1, Retinylpalmitat 0,3, | 2,2 |
| Alfalfa-Extrakt 0,8 und Meerfenchel-Extrakt | 1,0 |
| Dattelpalmenkern-Extrakt | 0,5 |
| Oligopeptid Matrikin/Ceramid 2 | 2,0 |
| RPF-Komplex¹ | 1,0 |

| **Phase D** | |
|---|---|
| Parfümöl | 0,5 |
| Konservierungsmittel | 0,3 |
| ¹ gemäß WO99/66881 Beispiel 1 | |

Die separat hergestellten Phasen werden nacheinander bei Raumtemperatur unter Rühren vermischt und homogenisiert.

### Beispiel 2 Gesichtscreme II

Die Zusammensetzung entsprach der des Beispiels 1, wobei das Oligopeptid Matrikin/Ceramid 2 nicht enthalten war.

### Beispiel 3 Gesichtscreme III

Die Zusammensetzung entsprach der des Beispiels 1, wobei das Oligopeptid Matrikin/Ceramid 2 sowie der RPF-Komplex nicht enthalten waren.

### Beispiel 4 Nachtcreme I

| **Phase A** | |
|---|---|
| Wasser | q.s. ad 100 |
| Sodium chloride | 1,0 |
| Glycerin | 4,0 |
| Butylenglycol | 2,0 |
| Pigmentgemisch Glimmerpigment und SiO₂-Pigment 1:1 | 0,8 |

| **Phase B** | |
|---|---|
| Cyclopentasiloxane /PEG 12 Dimethicone Crosspolymer | 10,0 |
| Cyclopentasiloxane/ Cyclotetrasiloxane | 10,0 |
| Dimethicone & Cyclotetrasiloxane | 3,0 |

| **Phase C** | |
|---|---|
| Wirkstoffkomplex bestehend aus Retinol (in Liposomen) 0,8, Retinylpalmitat 0,5, Alfalfa-Extrakt 2,0 und Meerfenchel-Extrakt 3,5 | 6,8 |

| **Phase D** | |
|---|---|
| Parfümöl | 0,5 |
| Konservierungsmittel | 0,3 |

Die separat hergestellten Phasen werden nacheinander bei Raumtemperatur unter Rühren vermischt und homogenisiert.

### Beispiel 5 Nachtcreme II

Die Zusammensetzung entsprach der des Beispiels 4, wobei zusätzlich 0,8 % Dattelpalmenkern-Extrakt enthalten waren.

### Beispiel 6 Nachtcreme III

Die Zusammensetzung entsprach der des Beispiels 4, wobei zusätzlich 0,7 % Dattelpalmenkern-Extrakt und 0,9 % RPF-Komplex (gemäß WO99/66881 Beispiel 1) enthalten waren.

### Beispiel 7 Körpercreme

| **Phase A** | |
|---|---|
| Wasser | q.s. ad 100 |
| Glycerin | 6,0 |
| Propylenglycol | 3,0 |
| Pigmentgemisch Glimmerpigment und SiO₂-Pigment 1:1 | 3,0 |

| **Phase B** | |
|---|---|
| Jojoba Oil | 6,0 |
| Tea Oil | 1,0 |
| Caffee Oil | 1,0 |

| **Phase C** | |
|---|---|
| Triethanolamin | 1,0 |

| **Phase D** | |
|---|---|
| Wirkstoffkomplex bestehend aus Retinol (in Liposomen) 0,3, Retinylpalmitat 0,5, Alfalfa-Extrakt 0,5 und Meerfenchel-Extrakt 5,0 | 6,3 |
| Oligopeptid Matrikin/Ceramid 2 | 0,5 |

| **Phase E** | |
|---|---|
| Parfümöl | 0,3 |
| Konservierungsmittel | 0,5 |

Die Phasen A und B werden jeweils separat auf etwa 75 °C erhitzt und dann unter Rühren zusammengeführt und ca. 20 Minuten homogenisiert. Danach wird mit der Phase C neutralisiert und dann auf maximal 40 °C abgekühlt. Es erfolgt anschließend die Zugabe der Phasen D und E unter Rühren und Homogenisieren.

### Beispiel 8 (Vergleichsbeispiel 1) Anwender-Test

Es wurde ein Anwendertest mit 80 Teilnehmerinnen im Alter von 36 bis 58 Jahren durchgeführt. 40 Teilnehmerinnen (Gruppe A1 und A2) applizierten zweimal täglich eine Creme gemäß Beispiel 4 im Gesicht, insbesondere rund um die Augenpartie. Die andere Gruppe (B1 und B2) applizierten eine Creme gemäß Beispiel 2 jedoch ohne die Komplexbestandteile Alfalfa-Extrakt und Meerfenchel-Extrakt. In den vorgegebenen Abständen von 2 Wochen wurden digitalisierte Aufnahmen insbesondere vom Augenwinkel der Teilnehmerinnen gemacht und über ein Computerprogramm nach Anzahl der Falten und Faltentiefe ausgewertet.

Jeweils 30 Teilnehmerinnen (Gruppe A1 und B1) führten den Test bis zu 12 Wochen bei zweimal täglicher Applikation zuende. Jeweils 10 Teilnehmerinnen (Gruppe A2 und B2) beendeten nach 8 Wochen die Applikation, wurden jedoch nach 10 und 12 Wochen nochmals untersucht.

Die Ergebnisse sind in Tabelle 1 dargestellt.

**Tabelle 1**

| | Faltenreduzierung [%] | | | |
|---|---|---|---|---|
| | Gruppe A1 | Gruppe B1 | Gruppe A2 | Gruppe B2 |
| Behandlungs-Beginn | 0 | 0 | 0 | 0 |
| nach 2 Wochen | 18-20 | 7-11 | 18-20 | 7-11 |
| 4 Wochen | 25-28 | 7-11 | 25-28 | 7-11 |
| 6 Wochen | 29-34 | 10-14 | 29-34 | 10-14 |
| 8 Wochen | 30-38 | 15-20 | 30 | 15 |
| 10 Wochen | 32-42 | 16-20 | 28-25 | 10-11 |
| 12 Wochen | 32-49 | 16-22 | 24-25 | 4-6 |

Tabelle 1 zeigt deutlich die Überlegenheit der Creme mit dem erfindungsgemäßen Komplex (A1, A2) gegenüber einer alleinigen Anwendung von Retinol/Retinylpalmitat (B1,B2). Während letztere (B1,B2) nach 6-8-wöchiger Anwendung auf einem Niveau von ca. 20% weitgehend stagnieren und bei Nichtanwendung in den Wochen danach nahezu auf den Ausgangswert zurückfallen (B2), ist der Erfolg der erfindungsgemäßen Creme bei der Faltenreduzierung etwa um das Doppelte höher, und darüber hinaus hält dieser Erfolg auch ohne weitere Anwendung noch mehrere Wochen an (A2).

In einer Nachfolgestudie wurde gefunden, dass bei Einsatz eines Mittels gemäß Beispiel 1 eine weitere Verbesserung um 5-9 % erreicht werden kann.

### Beispiel 9 (Vergleichsbeispiel 2)

In einem Test gemäß Beispiel 8 wurde eine Creme gemäß Beispiel 1 eingesetzt und bei einer Probandengruppe von 10 Teilnehmerinnen über einen Zeitraum von 10 Wochen angewandt. Die Faltenreduzierung lag bei 35-46 % und nach Absetzen der Creme nach insgesamt 12 Wochen noch bei 33-44 %.

## Patentansprüche

1. Pigmenthaltiges kosmetisches Mittel mit Anti-Alterungswirkung für die Haut, **dadurch gekennzeichnet, dass** es einen Wirkkomplex, Trägerstoffe, Hilfsstoffe und Gemische davon umfaßt, wobei der Wirkkomplex enthält
0,01 bis 5 Gew-% in Liposome verkapseltes Retinol,
0,1 bis 2 Gew-% Retinylpalmitat,
0,1 bis 3 Gew-% eines kohlehydratreichen, wässrigen Alfalfa-Extraktes,
0,1 bis 5 Gew-% eines Meerfenchel-Extraktes,
0,01 bis 5 Gew-% Schichtpigmente, ausgewählt unter Glimmer, beschichtet mit TiO₂/SiO₂ und einer weiteren TiO₂-Schicht; SiO₂-Plättchen, beschichtet mit Rutil-TiO₂;
und den Rest zu 100 Gew-% die Trägerstoffe oder Hilfsstoffe oder Gemische davon bilden, wobei alle Prozentangaben auf das Gesamtgewicht des kosmetischen Mittels bezogen sind.

2. Mittel nach Anspruch 1, **dadurch gekennzeichnet, dass** weitere Wirkstoffe enthalten sind.

3. Mittel nach Anspruch 2, **dadurch gekennzeichnet, dass** als weiterer Wirkstoff 0,1-2,5 Gew-% eines Dattelpalmenkernextraktes enthalten sind.

4. Mittel nach Anspruch 2, **dadurch gekennzeichnet, dass** als weiterer Wirkstoff 0,01-5 Gew-% einer synthetisch hergestellten Assoziation von Ceramid 2 mit dem palmitoylierten Oligopeptid Matrikin Pal-Val-Gly-Val-Ala-Pro-Gly enthalten sind.

5. Mittel nach Anspruch 2, **dadurch gekennzeichnet, dass** als weiterer Wirkstoff 0,1-2,5 Gew-% eines Dattelpalmenkernextraktes und 0,01-5 Gew-% einer synthetisch hergestellten Assoziation von Ceramid 2 mit dem palmitoylierten Oligopeptid Matrikin Pal-Val-Gly-Val-Ala-Pro-Gly enthalten sind.

6. Mittel nach einem der Ansprüche 1 bis 5, **dadurch gekennzeichnet, dass** als weiterer Wirkstoff 0,5-2 Gew-% einer Zubereitung mit einem Gehalt an einem durch Extraktion der Rinde von Quebracho blanco und nachfolgender enzymatischer Hydrolyse gewonnenem Produkt enthalten ist, das Proanthocyanidin-Oligomere enthält, in Mikrokapseln, sowie weiterhin einen durch Extraktion gewonnenen Seidenraupenextrakt, der das Peptid Cecropine, Aminosäuren und ein Vitamingemisch enthält, und ein Hydro-Gel oder Gemisch von Hydro-Gelen, und ein oder mehrere Phospholipide und Wasser.

7. Verwendung eines Mittels gemäß Anspruch 1 zur Anti-Falten-Behandlung der menschlichen Haut.

8. Verwendung eines Mittels gemäß Anspruch 5 zur Anti-Falten-Behandlung der menschlichen Haut.

## Claims

1. A cosmetic agent containing pigments with an anti-ageing effect for the skin, comprising an active complex, carrier substances, auxiliary substances and mixtures of these, wherein the active complex contains
0.01 to 5 weight percent retinol, encapsulated in liposomes,
0.1 to 2 weight percent retinyl palmitate,
0.1 to 3 weight percent of a carbohydrate-rich, aqueous alfalfa extract,
0.1 to 5 weight percent of a sea fennel extract,
0.01 to 5 weight percent layered pigments, selected under mica, coated with TiO₂/SiO₂ and a further TiO₂ layer; SiO₂ flakes, coated with rutile TiO₂;
and the carrier substances or auxiliary substances or mixtures of these form the remainder up to 100 weight percent, wherein all percentages given relate to the overall weight of the cosmetic agent.

2. An agent according to claim 1, wherein said agent contains further active substances.

3. An agent according to claim 2, wherein said agent contains as a further active substance 0.1-2.5 weight percentage of a date palm kernel extract.

4. An agent according to claim 2, wherein said agent contains as a further active substance 0.01-5 weight percentage of a synthetically produced association of ceramide 2 with the palmitoylated oligopeptide matrikin pal-val-gly-val-ala-pro-gly.

5. An agent according to claim 2, wherein said agent contains as a further active substance 0.1-2.5 weight percentage of a date palm kernel extract and 0.01-5 weight percent of a synthetically produced association of ceramide 2 with the palmitoylated oligopeptide matrikin pal-val-gly-val-ala-pro-gly.

6. An agent according to any one of claims 1 to 5, wherein said agent contains as a further active substance 0.5-2 weight percent of a preparation with a content of a product obtained by extracting the bark of quebracho blanco and a subsequent enzymatic hydrolysis, which contains proanthocyanidine oligomers, in micro-capsules, and furthermore a silkworm extract obtained by means of extraction, which contains the peptide cecropine, amino acids and a vitamin mixture, and a hydro-gel or a mixture of hydro-gels and one or more phospholipids and water.

7. The use of an agent according to claim 1 for the anti-wrinkle treatment of human skin.

8. The use of an agent according to claim 5 for the anti-wrinkle treatment of human skin.

## Revendications

1. Agent cosmétique contenant des pigments ayant une action antivieillissement pour la peau, **caractérisé en ce qu'**il contient un complexe actif, des excipients, des adjuvants et des mélanges de ces derniers, le complexe actif contenant
de 0,01 à 5 % en poids de rétinol capsulé dans des liposomes,
de 0,1 à 2 % en poids de palmitate de rétinyle,
de 0,1 à 3 % en poids d'un extrait d'alfalfa aqueux, riche en glucides,
0,1 à 5 % en poids d'un extrait de criste marine ;
de 0,01 à 5 % en poids de pigments en couche, sélectionnés parmi le mica, revêtu de TiO₂/SiO₂ et d'une autre couche de TiO₂ ; de plaquettes de SiO₂, revêtues de rutile TiO₂ ;
et le restant étant formé jusqu'à concurrence de 100 % en poids d'excipients ou d'adjuvants ou de mélanges de ces derniers, toutes les indications en pour cent se rapportant au poids total de l'agent cosmétique.

2. Agent selon la revendication 1, **caractérisée en ce que** d'autres principes actifs sont contenus.

3. Agent selon la revendication 2, **caractérisé en ce que** 0,1-2,5 % en poids d'un extrait de coeurs de palmiers dattiers sont contenus en tant que principe actif supplémentaire.

4. Agent selon la revendication 2, **caractérisé en ce que** 0,01-5 % en poids d'une association fabriquée synthétiquement de céramide 2 avec l'oligopeptide palmitoylé matrikin Pal-Val-Gly-Val-Ala-Pro-Gly sont contenus en tant que principe actif supplémentaire.

5. Agent selon la revendication 2, **caractérisé en ce que** 0,01-2,5 % en poids d'un extrait de coeurs de palmiers dattiers et de 0,01-5 % en poids d'une association fabriquée synthétiquement de céramide 2 avec l'oligopeptide palmitoylé matrikin Pal-Val-Gly-Val-Ala-Pro-Gly sont contenus en tant que principe actif supplémentaire.

6. Agent selon l'une quelconque des revendications 1 à 5, **caractérisé en ce que** 0,5-2 % en poids d'une préparation ayant une teneur en un produit obtenu par extraction de l'écorce de Quebracho blanco et par hydrolyse enzymatique subséquente, lequel produit contient des oligomères de la proanthocyanidine, dans des microcapsules, ainsi qu'en outre un extrait de vers à soie obtenu par extraction, lequel extrait contient le peptide cécropine, des aminoacides et un mélange de vitamines, et un hydrogel ou un mélange d'hydrogels, et un ou plusieurs phospholipides et de l'eau sont contenus en tant que principe actif supplémentaire.

7. Utilisation d'un agent selon la revendication 1 en vue du traitement antirides de la peau humaine.

8. Utilisation d'un agent selon la revendication 5 en vue du traitement antirides de la peau humaine.
